# EUROPEAN PATENT SPECIFICATION

(11) **EP 4 564 001 B1**
(45) Date of publication and mention of the grant of the patent: **11.11.2026**
(21) Application number: 23845920.0
(22) Date of filing: 11.04.2023
(51) Int. Cl.: G06T 7/00, G01N 33/48, C12M 1/34, G06T 1/00

(54) **ANALYSIS ASSISTANCE METHOD, PROGRAM, AND ANALYSIS ASSISTANCE DEVICE**
ANALYSEUNTERSTÜTZUNGSVERFAHREN, PROGRAMM UND ANALYSEUNTERSTÜTZUNGSVORRICHTUNG
PROCÉDÉ D'AIDE À L'ANALYSE, PROGRAMME ET DISPOSITIF D'AIDE À L'ANALYSE

(30) Priority: 26.07.2022 JP 2022118443
(43) Date of publication of application: 04.06.2025
(73) Proprietor: SCREEN Holdings Co., Ltd., Kyoto-shi, Kyoto 602-8585 (JP)
(72) Inventor: SHIBATA, Saya, Kyoto-shi, Kyoto 602-8585 (JP)
(74) Representative: Goddar, Heinz J.
(86) International application number: PCT/JP2023/014737
(87) International publication number: WO 2024/024180

(56) References cited:
- WO-A1-2017/150194
- CN-A- 111 583 226
- CN-A- 112 102 247
- CN-A- 112 927 188
- JP-A- 2002 165 757
- JP-A- 2008 185 337
- JP-A- 2008 535 528
- JP-A- 2011 095 225
- JP-A- 2012 073 179
- JP-A- 2018 529 074
- JP-A- 2020 154 952
- JP-A- 2020 173 204
- JP-A- 2021 061 836
- JP-A- 2022 123 488
- US-A1- 2019 080 450
- HAGHIGHAT MARYAM ET AL: "Automated quality assessment of large digitised histology cohorts by artificial intelligence", vol. 12, no. 1, 23 March 2022 (2022-03-23), US, XP093338775, ISSN: 2045-2322, Retrieved from the Internet <URL:https://www.nature.com/articles/s41598-022-08351-5> DOI: 10.1038/s41598-022-08351-5

## Description

### TECHNICAL FIELD

The present invention relates to a technique for supporting analysis of stained images of a sample.

### [CROSS-REFERENCE TO RELATED APPLICATIONS]

This application claims priority benefit of Japanese Patent Application No. JP2022-118443 filed In the Japan Patent Office on July 26, 2022.

### BACKGROUND ART

In recent year's research in medical or biological sciences, tissue-derived samples such as tissue samples or cell samples are analyzed in units of single cells for the purpose of, for example, elucidation of disease mechanisms, biological mechanisms, or drug action mechanisms. In multiplex immunostaining analysis, for example, biological products such as protein are stained by various methods, and stained conditions of the biological products are analyzed quantitatively and qualitatively. Japanese Unexamined Patent Application Publication (Translation of PCT Application) No. 2017-502362 (Document 1) proposes to align a plurality of digital images on a histological basis, the digital images being obtained by staining a tissue section of a patient by different types of staining methods.

In the aforementioned multiplex immunostaining analysis, stained images used for analysis may contain artifacts caused by staining methods (i.e., unintended staining results or fluctuations caused by factors such as the instability of staining). In this case, if a uniformly stained region is selected as an analytical field of view from a stained image, this stained image can also be used for analysis. On the other hand, the accuracy of analysis may decrease if artifacts are contained in the analytic field of view in even one stained image among a plurality of stained images that have undergone different types of staining. Accordingly, it is necessary to select a uniformly stained region as an analytic field of view for all of the stained images.

The aforementioned artifacts are, however, caused by a wide variety of factors. Thus, it is difficult to uniformly select an appropriate analytical field of view from each stained image, and under present circumstances, the analytic field of view is selected by skilled operators based on their experience. However, it is not easy even for skilled operators to select an appropriate analytic field of view in consideration of stained conditions of a plurality of stained images. Besides, the operators often enlarge the stained images for observation, which makes it difficult to visually check whether stained conditions are uniform.

CN 111 583 226 A discloses an analysis support method for supporting analysis of a stained image of a sample, the analysis support method comprising preparing a plurality of stained images by applying a plurality of types of stained methods on one sample, said plurality of stained images corresponding respectively to a plurality of types of staining methods, segmenting each stained image of said plurality of stained images into a plurality of segmented regions in a predetermined segmentation mode, and acquiring a uniformity level in said each stained image by using a trained model generated by machine learning, the uniformity level indicating uniformity of a stained condition of each of said plurality of segmented regions.

### SUMMARY OF THE INVENTION

The invention is defined by the appended claims.

The present invention is intended for an analysis support method for supporting analysis of stained images of a sample, and it is an object of the present invention to allow accurate presentation of an analysis matching region that is common among a plurality of stain images.

Aspect 1 of the present invention is an analysis support method according to claim 1.

The present invention allows accurate presentation of an analysis matching region that is common among the stained images.

Aspect 2 of the present invention is the analysis support method according to Aspect 1, in which an analysis matching region is displayed on the plurality of stained images in accordance with a result of the determination in the operation d), the analysis matching region being a segmented region that matches the analysis method.

Aspect 3 of the present invention is the analysis support method according to Aspect 2, in which a magnification of display of each of the plurality of stained images can be changed, and when the magnification of display of the each stained image is changed, a magnification of display of the analysis matching region on the each stained image is also changed together with the magnification of display of the each stained image.

Aspect 4 of the present invention is the analysis support method according to Aspect 1 (or according to any one of Aspects 1 to 3), in which an analysis unmatching region is displayed on the plurality of stained images in accordance with a result of the determination in the operation d), the analysis unmatching region being a segmented region that does not match the analysis method.

Aspect 5 of the present invention is the analysis support method according to Aspect 4, in which a magnification of display of each of the plurality of stained images can be changed, and when the magnification of display of the each stained image is changed, a magnification of display of the analysis unmatching region on the each stained image is also changed together with the magnification of display of the each stained image.

Aspect 6 of the present invention is the analysis support method according to Aspect 1 (or according to any one of Aspects 1 to 5), in which a middle region is displayed on the plurality of stained images in accordance with a result of the determination in the operation d), the middle region being a segmented region that may or may not match the analysis method.

Aspect 7 of the present invention is the analysis support method according to Aspect 6, in which a magnification of display of each of the plurality of stained images can be changed, and when the magnification of display of the each stained image is changed, a magnification of display of the middle region on the each stained image is also changed together with the magnification of display of the each stained image.

Aspect 8 of the present invention is the analysis support method according to any one of Aspects 1 to 7 that further includes, between the operations a) and c), performing resolution reduction processing on each of the plurality of stained images.

Aspect 9 of the present invention is the analysis support method according to Aspect 8, in which in each of the plurality of stained images that have undergone the resolution reduction processing, cells in the sample have an average diameter of greater than or equal to a pixel size.

Aspect 10 of the present invention is the analysis support method according to any one of Aspects 1 to 7 (or according to any one of Aspects 1 to 9), in which the trained model used to acquire the uniformity level in the operation c) is common among the plurality of stained images.

The present invention also provides a program for supporting analysis of a stained image of a sample. Aspect 11 of the present invention is a program according to claim 11.

The present invention also provides an analysis support device for supporting analysis of a stained image of a sample. Aspect 12 of the present invention is an analysis support device according to claim 12.

These and other objects, features, aspects and advantages of the present invention will become more apparent from the following detailed description of the present invention when taken in conjunction with the accompanying drawings.

### BRIEF DESCRIPTION OF DRAWINGS

Fig. 1 is a diagram showing a configuration of an analysis support device according to one embodiment.
Fig. 2 is a block diagram showing a function of the analysis support device.
Fig. 3 is a diagram showing one example of a stained image.
Fig. 4 is a diagram showing a plurality of segmented regions of a stained image.
Fig. 5 is an enlarged diagram showing one example of a learning image.
Fig. 6 is an enlarged diagram showing another example of the learning image.
Fig. 7 is a flowchart of processing performed by the analysis support device.
Fig. 8 is a diagram showing one example of displaying a class of each segmented region.
Fig. 9 is a diagram showing an image in which a mask is overlaid on a stained image.

### DESCRIPTION OF EMBODIMENTS

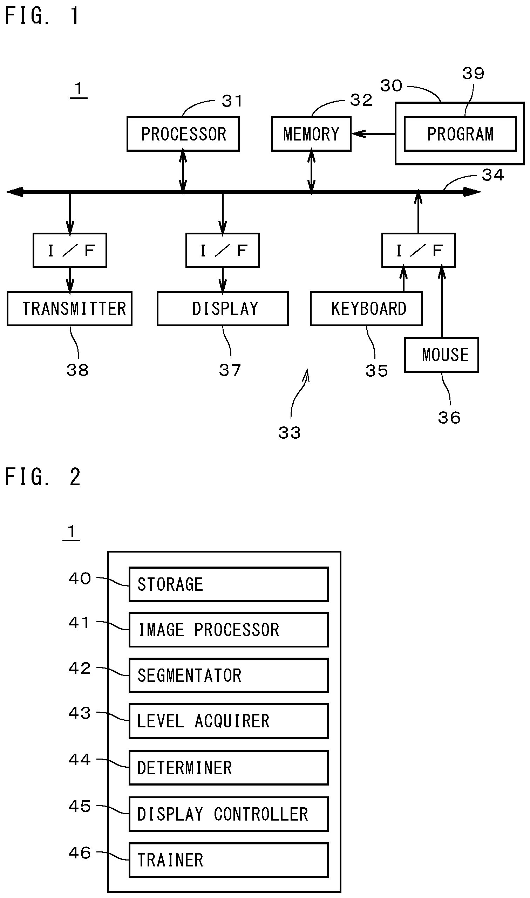

Fig. 1 is a diagram showing a configuration of a computer that functions as an analysis support device 1 according to one embodiment of the present invention. The analysis support device 1 is a device for supporting analysis of stained images of a sample and may be used for support of, for example, multiplex immunostaining analysis of a tissue-derived sample.

The analysis support device 1 is an ordinary computer that includes a processor 31, memory 32, an input/output device 33, and a bus 34. The bus 34 is a signal circuit that connects the processor 31, the memory 32, and the input/output device 33. The memory 32 stores various types of information. For example, the memory 32 may read out and store a program 39, which is a program product stored in advance in a storage medium 30. The storage medium 30 may, for example, be USB memory or a CD-ROM. The processor 31 executes a variety of processing (e.g., numerical calculation ) by using the memory 32 and so on in accordance with, for example, the aforementioned program 39 stored in the memory 32. The input/output device 33 includes a keyboard 35 and a mouse 36 that accept input from an operator, and a display 37 that displays, for example, output of the processor 31. The input/output device 33 further includes a transmitter 38 that transmits, for example, output of the processor 31.

Fig. 2 is a block diagram showing functions realized by the above-described computer executing the program 39, the computer configuring the analysis support device 1. As the functions realized by the computer, the analysis support device 1 includes a storage 40, an image processing unit 41, a segmentator 42, a level acquirer 43, a determiner 44, and a display controller 45. The storage 40 is mainly realized by the memory 32 (see Fig. 1) and stores data such as a plurality of stained images used for, for example, multiplex immunostaining analysis.

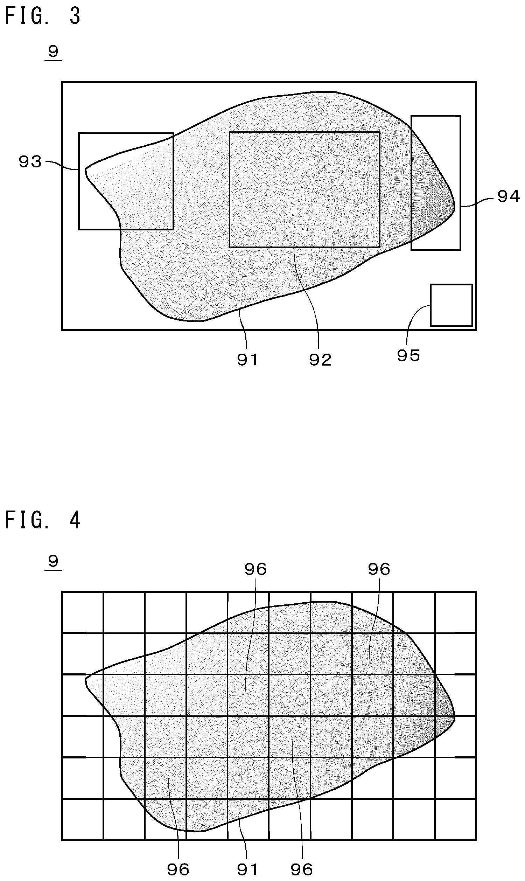

Fig. 3 is a diagram showing one example of a stained image 9 stored in the storage 40. The stained image 9 illustrated in Fig. 3 shows one stained sample 91 having an approximately rectangular shape. The sample 91 may, for example, be a tissue-derived sample that is analyzed by a predetermined analysis method such as multiplex immunostaining analysis. An approximately central region of the sample 91 in Fig. 3 (i.e., a region enclosed by a rectangular frame 92) is uniformly stained, so that the region 92 is suitable for analysis. Note that the region 92 is larger than the analytic field of view used for analysis of the stained image 9. In Fig. 3, a rectangle 95 shown in the lower right region of the stained image 9 represents the size of the analytic field of view.

An upper left region of the sample 91 in Fig. 3 (i.e., a region enclosed by a rectangular frame 93) is not stained uniformly and has gradations, but it has a relatively small change in concentration (concentration gradient) per unit distance. Thus, whether the region 93 is suitable for analysis or not depends on the type of analysis conducted using the stained image 9. A right end region of the sample 91 in Fig. 3 (i.e., a region enclosed by a rectangular frame 94) is not stained uniformly and has gradations, and it also has a relatively large concentration gradient. Thus, the region 94 is not suitable for analysis.

The image processing unit 41, the segmentator 42, the level acquirer 43, and the determiner 44 shown in Fig. 2 are mainly realized by the processor 31 (see Fig. 1). The image processing unit 41 performs pre-processing on the stained image 9, the pre-processing being for emphasizing whether the sample 91 is uniformly stained or not. This pre-processing may, for example, be resolution reduction processing.

The segmentator 42 segments the stained image 9 into a plurality of segmented regions in a predetermined segmentation mode. Fig. 4 is a diagram showing the stained image 9 segmented into a plurality of segmented regions 96. In the example shown in Fig. 4, the stained image 9 is segmented into a plurality of approximately rectangular segmented regions 96 arranged in longitudinal and lateral directions (i.e., arranged in a matrix). Each segmented region 96 may, for example, be a square with 256 pixels on each side. In Fig. 4, six segmented regions 96 are arranged in the longitudinal direction, and ten segmented regions 96 are arranged in the lateral direction. Note that the segmentation mode of the stained image 9 is not limited to the example shown in Fig. 4, and may be changed to any of various segmentation modes. For example, the stained image 9 may be segmented into approximately rectangular segmented regions that are different in size from the segmented regions 96 shown in Fig. 4. The shape of each segmented region 96 is not limited to an approximately rectangular shape, and may be changed to any of various shapes.

The level acquirer 43 shown in Fig. 2 acquires a uniformity level that indicates uniformity of the stained condition of each segmented region 96, by using a trained model generated by deep learning, which is one type of machine learning. In the case where a segmented region 96 has a uniform stained condition, like the aforementioned region 92, the uniformity level in the segmented region 96 may be set to "Level 0". In the case where a segmented region 96 has a gradation but has a small concentration gradient, like the aforementioned region 93, the uniformity level in the segmented region 96 may be set to "Level 1". In the case where a segmented region 96 has a gradation and a large concentration gradient, like the aforementioned region 94, the uniformity level in the segmented region 96 may be set to "Level 2". In the present example, as the numerical value for the level increases, the uniformity of the stained condition of each segmented region 96 decreases and suitability for analysis also decrease.

The aforementioned trained model may, for example, be generated in advance by a device other than the analysis support device 1 and stored in advance in the storage 40 of the analysis support device 1. The generation of the trained model is implemented by training an initial model with learning data by deep learning, the learning data indicating the relationship between each of a large number of learning images and the uniformity level, the learning images being obtained by capturing images of variously stained regions of various stained images 9. Examples of the learning images include images obtained by cutting out part of the stained image 9 of the sample 91 and images obtained by rotating or inverting the former images. The shapes and sizes of the cutout learning images may be the same as or different from the shapes and sizes of the segmented regions 96. The learning images may be acquired from a plurality of stained images 9 that correspond respectively to a plurality of types of staining methods used for analysis such as multiplex immunostaining analysis. The learning images may also be acquired from a plurality of stained images 9 that correspond respectively to a plurality of samples 91. The learning images may also be, for example, images other than the stained images 9 used for the aforementioned analysis. Note that the above-described trained model may be generated by machine learning other than deep learning.

As shown in Fig. 2, the analysis support device 1 may further include, as a function realized by the computer executing the program 39 (see Fig. 1), a trainer 46 that generates the above-described trained model. The trainer 46 is mainly realized by the processor 31. The trainer 46 generates the above-described trained model by subjecting an initial model to machine learning such as deep learning using the aforementioned large number pieces of learning data. The learning images used for the machine learning may, for example, be acquired in advance by an image capturing device provided within or independently of the analysis support device 1 and stored in the storage 40 of the analysis support device 1.

The learning data used for the machine learning performed by the trainer 46 is generated by an operator associating each learning image with a level that indicates the uniformity level of the learning image. For example, the operator may enlarge the learning images and compare stained conditions between adjacent positive and negative cells. Ordinarily, the positive cells are darkly stained, whereas the negative cells are lightly stained (or not stained). Thus, if there is a clear difference in concentration (i.e., a difference in luminance value) between adjacent positive and negative cells, it is determined that the stained condition is uniform, and the uniformity level of the stained condition of the learning image is set to Level 0.

On the other hand, in the case where the concentration of positive cells in a region where a learning image has a low concentration is lower than or approximately equivalent to the concentration of negative cells in a region where the learning image has a high concentration, it is determined that the stained condition of the learning image has gradations. Then, in the case where a region corresponding to the aforementioned analytic field of view 95 is cut out from the learning image and there are shadings in the region, it is determined that the gradations have a large concentration gradient, and the uniformity level of the stained condition of the learning image is set to Level 2. In the case where the cut-out region substantially has no gradations, it is determined that the gradations have a relatively small concentration gradient, and the uniformity level of the stained condition of the learning image is set to Level 1. In this way, the uniformity level of the stained condition of each learning image used for the generation of the above-described trained model is determined based on the concentrations of positive and negative cells (i.e., the luminance value) in the learning image.

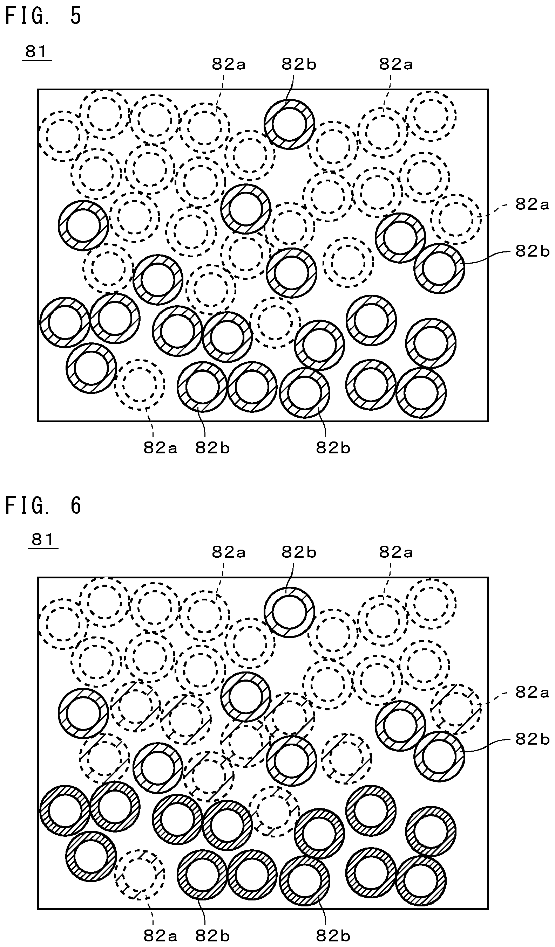

Fig. 5 is an enlarged diagram showing one example of a learning image 81 determined as Level 0. A large number of annular rings 82a and 82b in the learning image 81 each indicate a cell. In Fig. 5, the cells 82a illustrated with broken contours represent negative cells, and the cells 82b illustrated with solid contours represent positive cells. In the example shown in Fig. 5, the negative cells and the positive cells are normally stained. The positive cells are darkly stained, whereas the negative cells are lightly stained (or substantially not stained). In Fig. 5, the positive cells 82b are cross-hatched, and the negative cells 82a are not cross-hatched.

The upper portion of the learning image 81 illustrated in Fig. 5 includes densely spaced negative cells 82 and therefore appears lighter as a whole when the learning image 81 is not enlarged. A lower portion of the learning image 81 includes densely spaced positive cells 82b and therefore appears denser as a whole when the learning image 81 is not enlarged. A central portion of the learning image 81 in the up-down direction includes sparsely spaced positive cells 82b and therefore appears middle in concentration as a whole when the learning image 81 is not enlarged. Accordingly, when enlarged, the learning image 81 appears to have gradations, but when not enlarged, the learning image 81 has a clear difference in concentration between adjacent positive and negative cells 82b and 82a, so that the uniformity level of the stained condition of the learning image 81 is set to Level 0 as described above.

Fig. 6 is an enlarged diagram showing one example of a learning image 81 determined as Level 1 or 2. In Fig. 6, the densities of cross-hatching added to the negative cells 82a indicated by broken lines and to the positive cells 82b indicated by solid lines represent the concentrations of stained cells, and a higher cross-hatching density indicates a higher concentration. The upper portion of the learning image 81 illustrated in Fig. 6 is a light-colored region with insufficient staining, and the lower portion of the learning image 81 is a dark-colored region with excessive staining. Thus, the learning image 81 illustrated in Fig. 6 has gradations when not enlarged. In the learning image 81 illustrated in Fig. 6, the concentration of positive cells 82b in the upper light-colored region is approximately equivalent to the concentration of negative cells 82a in the lower dark-colored region. Therefore, the learning image 81 is determined to actually have gradations, and the uniformity level of the stained condition of the learning image 81 is set to Level 1 or 2.

The determiner 44 determines suitability of each of the segmented regions 96 shown in Fig. 4 for a predetermined analysis method such as multiplex immunostaining analysis in accordance with the uniformity level of the stained condition acquired by the level acquirer 43. The display controller 45 displays the result of the determination by the determiner 44 on the display 37.

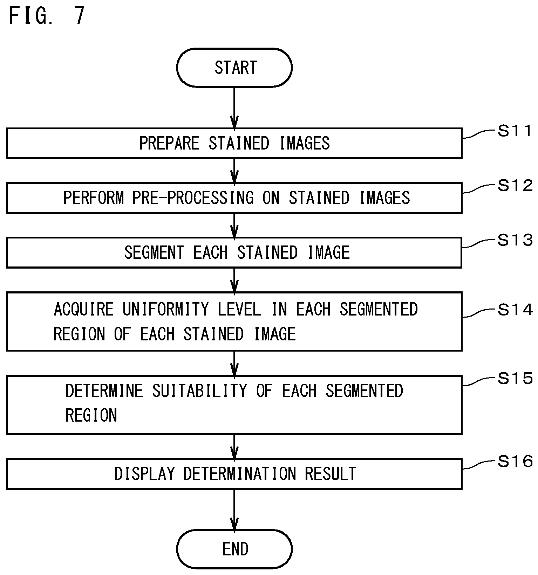

Next, a procedure of processing performed by the analysis support device 1 is described with reference to Fig. 7. The analysis support device 1 firstly prepares a plurality of stained images 9 by storing them in the storage 40 (step S11). The stained images 9 are obtained by capturing an image of a sample 91 by applying a plurality of types (e.g., nine types) of staining methods corresponding to a predetermined analysis method (e.g., multiplex immunostaining analysis) on the sample 91. The plurality of stained images 9 correspond respectively to the aforementioned plurality of types of staining methods. The sample 91 may, for example, be a slide glass sample. The thickness of the sample 91 may, for example, be less than or equal to 4 µm. Each stained image 9 may be obtained by, for example, capturing an image of one immune-stained sample 91 as a whole. The resolution of the stained image 9 may, for example, be approximately 0.46 µm per pixel. The diameter of cells included in the sample 91 in each stained image 9 is approximately in the range of 10 to 30 pixels.

Then, the image processing unit 41 performs the aforementioned pre-processing on each of the stained images 9 (step S12). In step S12, the pre-processing is performed on the stained image 9 so that the boundaries of individual cells in the stained image 9 become ambiguous. It is, however, noted that the pre-processing is performed within such a range that allows an operator to visually recognize the presence or absence of individual cells in order to allow determination as to whether a dark-colored region in the stained image 9 is either a group of positive cells 82b (see Fig. 5) or a dark-colored region with excessive staining (see Fig. 6).

The pre-processing may, for example, be resolution reduction processing for reducing the resolution of each stained image 9. In the resolution reduction processing, for example, each stained image 9 may be processed such that the resolution of the stained image 9 is reduced to approximately one fourth. In each of the stained images 9 that have undergone the resolution reduction processing, the average diameter of the cells (i.e., the negative cells and the positive cells) in the sample 91 may preferably be greater than or equal to a pixel size. The pixel size as used herein refers to the length of each side of one pixel in the stained images 9 that have undergone the resolution reduction processing. The average diameter of the cells also refers to an arithmetical mean of the diameters of all the cells in the sample 91. Note that the above-described pre-processing is not limited to the resolution reduction processing, and may be changed to any other processing. For example, blurring processing using a filter or the like may be performed as the pre-processing on each stained image 9.

Then, the segmentator 42 segments each of the stained images 9 into a plurality of segmented regions 96 (see Fig. 4) in a predetermined segmentation mode (step S13). The same segmentation mode is used for the stained images 9, and the shape and positions of the segmented regions 96 in one stained image 9 are the same as those in the other stained images 9. Note that step S13 may be performed in parallel with step S12 described above, or may be performed between steps S11 and S12. That is, steps S12 and S13 are performed between step S11 and step S14, which will be described later.

When steps S11 to S13 end, the level acquirer 43 acquires, for each segmented region 96, the uniformity level indicating uniformity of the stained condition of the segmented region 96 (step S14). The acquisition of the uniformity level in each segmented region 96 by the level acquirer 43 is conducted using a trained model generated by machine learning such as deep learning as described above. Accordingly, it is possible to accurately acquire the uniformity level in each segmented region 96 without being affected by an factor such as operator's proficiency.

In step S14, for example, a common trained model is used to acquire the uniformity level in each of the stained images 9. In this case, the learning images used for the generation of the trained model may include, for example, images acquired from each of the aforementioned stained images 9. Using a common trained model among the stained images 9 allows more speedy execution of step S14 than in cases such as where the trained model is changed for each type of stained images 9.

Then, the determiner 44 determines suitability of each of the segmented regions 96 illustrated in Fig. 4 for the aforementioned predetermined method (step S15). In step S15, whether each of the segmented regions 96 is suitable for use in the predetermined analysis method is determined based on the uniformity level acquired for the stained image 9.

Specifically, for one segmented region 96, the determiner 44 confirms the uniformity levels acquired for the stained images 9. Then, for example, in the case where the uniformity levels in all of the stained images 9 are Level 0, the one segmented region 96 is classified into "Class 0," which indicates that the segmented region is an analysis matching region that has a uniform stained condition in all of the stained images 9 and that matches the aforementioned predetermined analysis method. In the case where one or more of the uniformity levels acquired for the stained images 9 are Level 2, the one segmented region 96 is classified into "Class 2," which indicates that the segmented region is an analysis unmatching region that has a non-uniform stained condition and that does not match the aforementioned predetermined analysis method. Meanwhile, in the case where one or more of the uniformity levels acquired for the stained images 9 are Level 1 but none of them is Level 2, the one segmented region 96 is classified into "Class 1," which indicates that the segmented region is a middle region that has a slightly non-uniform stained condition and that may or may not match the aforementioned predetermined analysis method.

In step S15, the aforementioned class classification is conducted in sequence on the segmented regions 96, and the class is determined for each segmented region 96. Note that the criterion for the aforementioned class classification may be changed in various ways. For example, among the uniformity levels acquired for the stained images 9 for one segmented region 96, if the number of uniformity levels indicating Level 2 is zero and the number of uniformity levels indicating Level 1 is one or less, the one segmented region 96 may be classified into Class 0. If the number of uniformity levels indicating Level 2 is one or more, the one segmented region 96 may be classified into Class 1. In the other cases, the one segmented region 96 may be classified into Class 2.

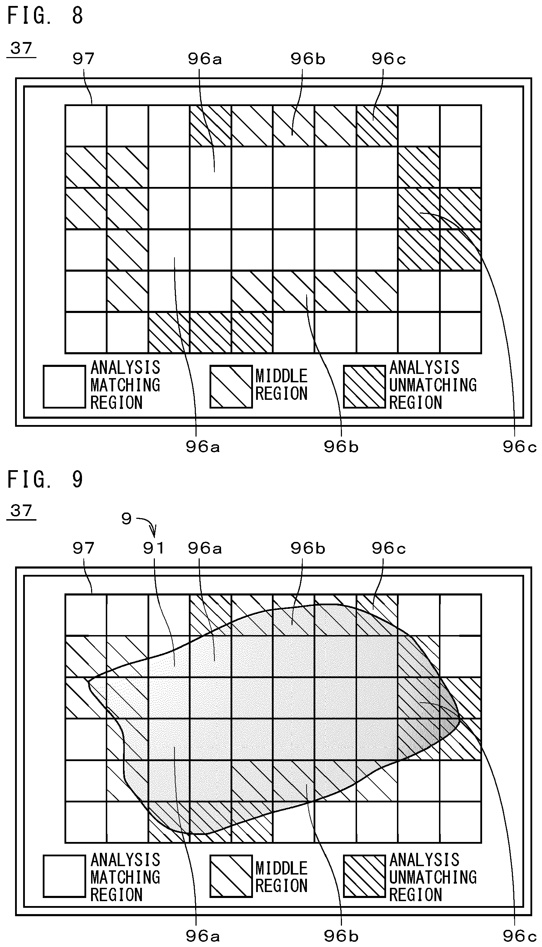

When step S15 ends, the display controller 45 displays the class of each segmented region 96 on the display 37 in accordance with the result of the determination in step S15 (step S16). Fig. 8 is a diagram showing one example of displaying the class of each segmented region on the display 37. In Fig. 8, the analysis matching regions 96a that are segmented regions matching the aforementioned predetermined analysis method (i.e., segmented regions 96 classified into Class 0) are illustrated with no cross-hatching. The analysis unmatching regions 96c that do not match the predetermined analysis method (i.e., segmented regions 96 classified into Class 2) are illustrated with relatively dense cross-hatching. The middle regions 96b that may or may not match the predetermined analysis method (i.e., segmented regions 96 classified into Class 1) are illustrated with relatively sparse cross-hatching as compared with the analysis unmatching regions 96c. This enables the operator to easily recognize regions of a stained image 9 that are suitable for analysis, regions that are not suitable for analysis, and regions that may be or may not be suitable for analysis. In the following description, an image of the segmented regions 96 with each class visibly displayed is also referred to as a "mask 97." In the example shown in Fig. 8, explanatory legends are displayed below the mask 97 (the same applies to Fig. 9).

Fig. 9 shows the mask 97 displayed (i.e., overlaid) over one stained image 9 on the display 37. This enables the operator to more easily recognize regions of the stained image 9 that are suitable for analysis, regions that are not suitable for the analysis, and regions that may be or may not be suitable for analysis.

In the stained image 9 shown in Fig. 9, the sample 91 is most difficult to see in regions that overlap with the analysis unmatching regions 96c, and the sample 91 is most easy to see in regions that overlap with the analysis matching region 96a. In regions of the stained image 9 that overlap with the middle regions 96b, the sample 91 is more easy to see than in the regions overlapping with the analysis unmatching regions 96c and is more difficult to see than in the regions overlapping with the analysis matching regions 96a. In other words, the analysis unmatching regions 96c of the stained image 9 are masked to make them most difficult to see, and the analysis matching regions 96a are extracted from the segmented regions 96 so as to make them more easy to see. Accordingly, when the operator sets the analytic field of view 95 (see Fig. 3) on a stained image 9 for analysis using the stained image 9, the analytic field of view 95 can be easily set on an analysis matching region 96a.

The analysis support device 1 is capable of displaying the analysis matching regions 96a, the middle regions 96b, and the analysis unmatching regions 96c over each of the aforementioned stained images 9. On the display 37, one stained image 9 may be displayed on which the analysis matching regions 96a, the middle regions 96b, and the analysis unmatching regions 96c are overlaid, or two or more stained images 9 may be displayed at the same time, on each of which the analysis matching regions 96a, the middle regions 96b, and the analysis unmatching regions 96c are overlaid.

On the actual display 37, the analysis matching regions 96a, the middle regions 96b, and the analysis unmatching regions 96c may be distinguished by different cross-hatching techniques as shown in Fig. 8, or may be distinguished by different coloring. As another alternative, the analysis matching regions 96a, the middle regions 96b, and the analysis unmatching regions 96c may be distinguished on the display 37 by any of various techniques other than cross-hatching or coloring.

In the analysis support device 1, the display controller 45 (see Fig. 2) is capable of changing the magnification of display of each stained image 9 displayed on the display 37. In the case of changing the magnification of display of each stained image 9, the display controller 45 also changes the magnification of display of the analysis matching regions 96a, the middle regions 96b and the analysis unmatching regions 96c of the stained image 9 together with the magnification of display of the stained image 9.

In step S16, the mask 97 does not necessarily have to be displayed over the stained image 9, and the mask 97 alone may be displayed on the display 37 as shown in Fig. 8. In step S16, only one or two types of regions among the analysis matching regions 96a, the middle regions 96b, and the analysis unmatching regions 96c may be selectively displayed over or not over the stained image 9 on the stained image 9.

As another alternative, another mask different from the mask 97 may be displayed over or not over the stained image 9 on the display 37. For example, a mask that indicates the uniformity level in each segmented region 96 of one stained image 9 may be displayed over the stained image 9. For example, this mask may add no cross-hatching to the segmented regions 96 classified into Level 0, add relatively dense cross-hatching to segmented regions 96 classified into Level 2, and add relatively sparse cross-hatching to segmented regions 96 classified into Level 1. This allows regions that are suitable for analysis, regions that are not suitable for analysis, and regions that may be or may not be suitable for analysis in the stained image 9 to be presented to the operator in an easy-to-understand manner.

In the aforementioned analysis support method for use in the analysis support device 1, each segmented region 96 may be classified into either Class 0 or Class 2 in step S15, with Class 1 omitted. For example, for one segmented region 96, in the case where all of the uniformity levels acquired for the stained images 9 are Level 0, the one segmented region 96 may be classified into Class 0 indicating the analysis matching region, and otherwise the one segmented region 96 may be classified into Class 2 indicating the analysis unmatching region. The criterion for the class classification may be changed in various ways. For example, among the uniformity levels acquired for one segmented region 96 of the stained images 9, if the number of uniformity levels indicating Level 2 is zero and the number of uniformity levels indicating Level 1 is two or less, the one segmented region 96 may be classified into Class 0, and otherwise the one segmented region 96 may be classified into Class 2.

As described above, the aforementioned analysis support method for supporting the analysis of the stained images 9 of the sample 91 includes the step of preparing a plurality of stained images 9 by applying a plurality of types of staining methods on one sample 91, the plurality of stained images 9 corresponding respectively to the plurality of types of staining methods (step S11), the step of segmenting each of the stained images 9 into a plurality of segmented regions 96 in a predetermined segmentation mode (step S13), the step of acquiring the uniformity level in each stained image 9 by using a trained model generated by machine learning, the uniformity level indicating uniformity of the stained condition of each of the segmented regions 96 (step S14), and the step of determining suitability of each of the segmented regions 96 for a predetermined analysis method in accordance with the uniformity level in each of the stained images 9.

This allows, as described above, accurate acquisition of the uniformity level of the stained condition for each segmented region 96 of the stained images 9. Accordingly, it is possible to accurately determine suitability of each segmented region 96 for analysis. As a result, it is possible to accurately present an analysis matching region that is common among the stained images 9.

As described above, in the analysis support method, it is preferable that the analysis matching regions 96a, which are the segmented regions 96 matching the aforementioned analysis method, may be displayed over the stained images 9 in accordance with the result of the determination in step S15. This allows the operator or the like who conducts analysis to easily recognize a region suitable for the analysis of the stained images 9 and to easily set this region as the analytic field of view 95. As a result, it is possible to improve the accuracy of analysis using the stained images 9.

As described above, in the analysis support method, it is preferable that the magnification of display of each of the stained images 9 can be changed, and when the magnification of display of each stained image 9 is changed, the magnification of display of the analysis matching regions 96a over the stained image 9 may also be changed together with the magnification of display of the stained image 9. This allows the operator or the like to easily recognize a region suitable for the analysis of the stained images 9 even in the case where the magnification of display of the stained images 9 has been changed.

As described above, in the analysis support method, it is preferable that the analysis unmatching regions 96c, which are the segmented regions 96 that do not match the aforementioned analysis method, may be displayed over the stained images 9 in accordance with the result of the determination in step S15. This allows the operator or the like who conducts analysis to easily recognize regions that do not match the analysis of the stained images 9 and to easily set the analytic field of view 95 while avoiding the unmatching regions. As a result, it is possible to improve the accuracy of analysis using the stained images 9.

As described above, in the analysis support method, it is preferable that the magnification of display of the stained images 9 can be changed, and when the magnification of display of each stained image 9 is changed, the magnification of display of the analysis unmatching regions 96c over the stained image 9 may also be changed together with the magnification of display of the stained image 9. This allows the operator or the like to easily recognize regions that do not match the analysis of the stained images 9 even in the case where the magnification of display of the stained images 9 has been changed.

As described above, in the analysis support method, it is preferable that the middle regions 96b, which are the segmented regions 96 that may or may not match the above-described analysis method, may be displayed over the stained images 9 in accordance with the result of the determination in step S15. This enables the operator or the like who conducts analysis to easily recognize regions that may or may not match the analysis of the stained images 9. Thus, in the case where analysis capable of using these regions is conducted, it is possible to enlarge the range of the stained images 9 within which the analytic field of view 95 is set. As a result, it is possible to improve the degree of freedom in analysis. In the case where analysis incapable of using these regions is conducted, it is possible to easily set the analytic field of view 95 while avoiding these regions. As a result, it is possible to improve the accuracy of analysis.

As described above, in the analysis support method, it is preferable that the magnification of display of each of the stained images 9 can be changed, and when the magnification of display of each stained image 9 is changed, the magnification of display of the middle regions 96b on the stained image 9 may also be changed together with the magnification of display of the stained image 9. This allows the operator or the like to easily recognize regions that may or may not match the analysis of the stained images 9 even in the case where the magnification of display of the stained images 9 has been changed.

As described above, it is preferable that the analysis support method further includes the step of performing the resolution reduction processing on each of the stained images 9 (step S12) between steps S11 and S14. This allows accurate acquisition of the uniformity level in step S14.

As described above, it is preferable that, in each of the stained images 9 that have undergone the resolution reduction processing, the average diameter of the cells in the sample 91 may be greater than or equal to the pixel size. By performing the resolution reduction processing to the extent that the cells can be identified individually, it is possible to favorably acquire the uniformity level in step S14.

As described above, it is preferable that a common trained model may be used to acquire the uniformity level in each of the stained images 9 in step S14. This allows more speedy execution of step S14 than in cases such as where the trained model is changed for each type of stained images 9. It may also become possible to reduce time and effort required for the generation of trained models corresponding to the stained images 9.

As described above, the uniformity level in each learning image 81 used for the generation of the trained model is determined based on the luminance values of the positive cells 82b and the negative cells 82a in the learning image 81. This allows the trained model to be generated favorably.

As described above, the computer executes the program 39 to perform the step of preparing a plurality of stained images 9 by applying a plurality of types of staining methods on one sample 91, the plurality of stained images corresponding respectively to the plurality of types of staining methods (step S11), the step of segmenting each stained image 9 of the stained images 9 into a plurality of segmented regions 96 in a predetermined segmentation mode (step S13), the step of acquiring the uniformity level in each stained image by using a trained model generated by machine learning, the uniformity level indicating uniformity of the stained condition of each of the segmented regions 96 (step S14), and the step of determining suitability of each segmented region 96 of the segmented regions 96 for a predetermined analysis method in accordance with the uniformity level in each of the stained images 9 (step S15). This allows, as described above, accurate presentation of analysis matching regions that are common among the stained images 9.

The aforementioned analysis support device 1 includes the segmentator 42 that segments each stained image 9 of the stained images 9 into a plurality of segmented regions 96 in a predetermined segmentation mode, the stained images being obtained by applying a plurality of types of staining methods on one sample 91 the stained images corresponding respectively to the plurality of types of staining methods, the level acquirer 43 that acquires the uniformity level in each stained image 9 by using a trained model generated by machine learning, the uniformity level indicating uniformity of the stained condition of each of the segmented region 96, and the determiner 44 that determines suitability of each segmented region 96 of the segmented regions 96 for a predetermined analysis method in accordance with the uniformity level in each of the stained images 9. This allows, as described above, accurate presentation of analysis matching regions that are common among the stained images 9.

The analysis support method, the program 39, and the analysis support device 1 described above may be modified in various ways.

In step S14, a common trained model does not necessarily have to be used to acquire the uniformity level in each of the stained images 9. For example, a dedicated trained model may be used for each of the stained images 9. As another alternative, a common trained model may be used for only two or more of the stained images 9.

In the above-described example, the class indicating the suitability of each segmented region for analysis is divided into three (0, 1, and 2), but the class may be divided into a smaller number of classes. For example, in the case where the number of stained images 9 is nine, if all the uniformity levels acquired for one segmented region 96 in the stained images 9 indicate Level 0, the one segmented region 96 may be classified into Class 0. If, among the uniformity levels acquired for the stained images 9, the number of uniformity levels indicating Level 2 are zero and the number of uniformity levels indicating Level 1 is one, the one segmented region 96 may be classified into "Class 0.2." If the number of uniformity levels indicating Level 2 is zero and the number of uniformity levels indicating Level 1 is two, the one segmented region 96 may be classified into "Class 0.4." If the number of uniformity levels indicating Level 2 is zero and the number of uniformity levels indicating Level 1 is three, the one segmented region 96 may be classified into "Class 0.6." If the number of uniformity levels indicating Level 2 is zero and the number of uniformity levels indicating Level 1 is four, the one segmented region 96 may be classified into "Class 0.8." If, among the uniformity levels acquired for the stained images 9, the number of uniformity levels indicating Level 2 is zero and the number of uniformity levels indicating Level 1 is five or more, the one segmented region 96 may be classified into Class 1. If the number of uniformity levels indicating Level 2 is one or more, the one segmented region 96 may be classified into Class 2.

In step S12, in each of the stained images 9 that have undergone the resolution reduction processing, the average diameter of the cells in the sample 91 may be less than the pixel size. Moreover, as described above, the pre-processing other than the resolution reduction processing may be performed, instead of or in addition to the resolution reduction processing in step S12.

The analysis support method, the program 39, and the analysis support device 1 described above do not necessarily have to be used for the support of multiplex immunostaining analysis, and may be used for the support of any of a variety of analysis other than multiplex immunostaining analysis. The sample 91 does not necessarily have to be a tissue-derived sample, and may be any other sample.

The configurations of the above-described preferred embodiments and variations may be appropriately combined as long as there are no mutual inconsistencies.

While the invention has been shown and described in detail, the foregoing description is in all aspects illustrative and not restrictive. It is therefore understood that numerous modifications and variations can be devised without departing from the scope of the invention.

### REFERENCE SIGNS LIST

- 1: analysis support device
- 9: stained image
- 39: program
- 42: segmentator
- 43: level acquirer
- 44: determiner
- 81: learning image
- 82a: negative cell
- 82b: positive cell
- 91: sample
- 96: segmented region
- 96a: analysis matching region
- 96b: middle region
- 96c: analysis unmatching region
- S11 to S16: step

## Claims

1. An analysis support method for supporting analysis of a stained image (9) of a sample (91), the analysis support method comprising:
a) preparing a plurality of stained images (9) by applying a plurality of types of staining methods on one sample (91), said plurality of stained images (9) corresponding respectively to said plurality of types of staining methods (S11);
b) segmenting each stained image (9) of said plurality of stained images (9) into a plurality of segmented regions (96) in a predetermined segmentation mode (S13);
c) acquiring a uniformity level in said each stained image (9) by using a trained model generated by machine learning, the uniformity level indicating uniformity of a stained condition of each of said plurality of segmented regions (96) (S14); and
d) determining suitability of each segmented region (96) of said plurality of segmented regions (96) for a predetermined analysis method in accordance with said uniformity level in each of said plurality of stained images (9) (S15), wherein
said uniformity level in a learning image (81) that is used to generate said trained model is determined based on luminance values of a positive cell (82b) and a negative cell (82a) in said learning image (81)

2. The analysis support method according to claim 1, wherein
an analysis matching region (96a) is displayed on said plurality of stained images (9) in accordance with a result of the determination in said operation d) (S15), the analysis matching region (96a) being a segmented region (96) that matches said analysis method.

3. The analysis support method according to claim 2, wherein
a magnification of display of each of said plurality of stained images (9) can be changed, and
when the magnification of display of said each stained image (9) is changed, a magnification of display of said analysis matching region (96a) on said each stained image (9) is also changed together with the magnification of display of said each stained image (9).

4. The analysis support method according to any one of claims 1 to 3, wherein
an analysis unmatching region (96c) is displayed on said plurality of stained images (9) in accordance with a result of the determination in said operation d) (S15), the analysis unmatching region (96c) being a segmented region (96) that does not match said analysis method.

5. The analysis support method according to claim 4, wherein
a magnification of display of each of said plurality of stained images (9) can be changed, and
when the magnification of display of said each stained image (9) is changed, a magnification of display of said analysis unmatching region (96c) on said each stained image (9) is also changed together with the magnification of display of said each stained image (9).

6. The analysis support method according to any one of claims 1 to 5, wherein
a middle region (96b) is displayed on said plurality of stained images (9) in accordance with a result of the determination in said operation d) (S15), the middle region (96b) being a segmented region (96) that may or may not match said analysis method.

7. The analysis support method according to claim 6, wherein
a magnification of display of each of said plurality of stained images (9) can be changed, and
when the magnification of display of said each stained image (9) is changed, a magnification of display of said middle region (96b) on said each stained image (9) is also changed together with the magnification of display of said each stained image (9).

8. The analysis support method according to any one of claims 1 to 7, further comprising:
between said operations a) and c) (S11, S14), performing resolution reduction processing on each of said plurality of stained images (9) (S12).

9. The analysis support method according to claim 8, wherein
in each of said plurality of stained images (9) that have undergone said resolution reduction processing, cells (82a, 82b) in said sample (91) have an average diameter of greater than or equal to a pixel size.

10. The analysis support method according to any one of claims 1 to 9, wherein
said trained model used to acquire said uniformity level in said operation c) (S14) is common among said plurality of stained images (9).

11. A program (39) for supporting analysis of a stained image (9) of a sample (91),
said program (39) being executed by a computer to:
a) prepare a plurality of stained images (9) by applying a plurality of types of staining methods on one sample (91), said plurality of stained images (9) corresponding respectively to said plurality of types of staining methods (S11);
b) segment each stained image (9) of said plurality of stained images (9) into a plurality of segmented regions (96) in a predetermined segmentation mode (S13);
c) acquire a uniformity level in said each stained image (9) by using a trained model generated by machine learning, the uniformity level indicating uniformity of a stained condition of each of said plurality of segmented regions (96) (S14); and
d) determine suitability of each segmented region (96) of said plurality of segmented regions (96) for a predetermined analysis method in accordance with said uniformity level in each of said plurality of stained images (9) (S15), wherein
said uniformity level in a learning image (81) that is used to generate said trained model is determined based on luminance values of a positive cell (82b) and a negative cell (82a) in said learning image (81).

12. An analysis support device (1) for supporting analysis of a stained image (9) of a sample (91), the analysis support device comprising:
a segmentator (42) that segments each stained image (9) of a plurality of stained images (9) into a plurality of segmented regions (96) in a predetermined segmentation mode, said plurality of stained images (9) being obtained by applying a plurality of types of staining methods on one sample (91), said plurality of stained images (9) corresponding respectively to said plurality of types of staining methods;
a level acquirer (43) that acquires a uniformity level in said each stained image (9) by using a trained model generated by machine learning, the uniformity level indicating uniformity of a stained condition of each of said plurality of segmented regions (96); and
a determiner (44) that determines suitability of each segmented region (96) of said plurality of segmented regions (96) for a predetermined analysis method in accordance with said uniformity level in each of said plurality of stained images (9), wherein
said uniformity level in a learning image (81) that is used to generate said trained model is determined based on luminance values of a positive cell (82b) and a negative cell (82a) in said learning image (81).

## Patentansprüche

1. Analyseunterstützungsverfahren zum Unterstützen der Analyse eines gefärbten Bildes (9) einer Probe (91), wobei das Analyseunterstützungsverfahren umfasst:
a) Vorbereiten einer Vielzahl von gefärbten Bildern (9) durch Anwenden einer Vielzahl von Typen von Färbeverfahren auf eine Probe (91), wobei die Vielzahl von gefärbten Bildern (9) jeweils der Vielzahl von Typen von Färbeverfahren entspricht (S11);
b) Segmentieren jedes gefärbten Bildes (9) der Vielzahl von gefärbten Bildern (9) in eine Vielzahl von segmentierten Regionen (96) in einem vorbestimmten Segmentierungsmodus (S13);
c) Erfassen eines Gleichförmigkeitsniveaus in jedem gefärbten Bild (9) unter Verwendung eines trainierten Modells, das durch maschinelles Lernen erzeugt wird, wobei das Gleichförmigkeitsniveau die Gleichförmigkeit eines gefärbten Zustands jeder der Vielzahl von segmentierten Regionen (96) angibt (S14); und
d) Bestimmen der Eignung jeder segmentierten Region (96) der Vielzahl von segmentierten Regionen (96) für ein vorbestimmtes Analyseverfahren gemäß dem Gleichförmigkeitsniveau in jedem der Vielzahl von gefärbten Bildern (9) (S15), wobei
das Gleichförmigkeitsniveau in einem Lernbild (81), das zum Erzeugen des trainierten Modells verwendet wird, basierend auf Leuchtdichtewerten einer positiven Zelle (82b) und einer negativen Zelle (82a) in dem Lernbild (81) bestimmt wird.

2. Analyseunterstützungsverfahren nach Anspruch 1, wobei
eine Analyseübereinstimmungsregion (96a) auf der Vielzahl von gefärbten Bildern (9) gemäß einem Ergebnis der Bestimmung in der Operation d) angezeigt wird (S15), wobei die Analyseübereinstimmungsregion (96a) eine segmentierte Region (96) ist, die mit dem Analyseverfahren übereinstimmt.

3. Analyseunterstützungsverfahren nach Anspruch 2, wobei
eine Vergrößerung der Anzeige jedes der Vielzahl von gefärbten Bildern (9) geändert werden kann, und
wenn die Vergrößerung der Anzeige jedes gefärbten Bildes (9) geändert wird, eine Vergrößerung der Anzeige der Analyseübereinstimmungsregion (96a) auf jedem gefärbten Bild (9) auch zusammen mit der Vergrößerung der Anzeige jedes gefärbten Bildes (9) geändert wird.

4. Analyseunterstützungsverfahren nach einem der Ansprüche 1 bis 3, wobei
eine Analysenichtübereinstimmungsregion (96c) auf der Vielzahl von gefärbten Bildern (9) gemäß einem Ergebnis der Bestimmung in der Operation d) angezeigt wird (S15), wobei die Analysenichtübereinstimmungsregion (96c) eine segmentierte Region (96) ist, die nicht mit dem Analyseverfahren übereinstimmt.

5. Analyseunterstützungsverfahren nach Anspruch 4, wobei
eine Vergrößerung der Anzeige jedes der Vielzahl von gefärbten Bildern (9) geändert werden kann, und
wenn die Vergrößerung der Anzeige jedes gefärbten Bildes (9) geändert wird, eine Vergrößerung der Anzeige der Analysenichtübereinstimmungsregion (96c) auf jedem gefärbten Bild (9) auch zusammen mit der Vergrößerung der Anzeige jedes gefärbten Bildes (9) geändert wird.

6. Analyseunterstützungsverfahren nach einem der Ansprüche 1 bis 5, wobei
eine mittlere Region (96b) auf der Vielzahl von gefärbten Bildern (9) gemäß einem Ergebnis der Bestimmung in der Operation d) angezeigt wird (S15), wobei die mittlere Region (96b) eine segmentierte Region (96) ist, die mit dem Analyseverfahren übereinstimmen kann oder nicht.

7. Analyseunterstützungsverfahren nach Anspruch 6, wobei
eine Vergrößerung der Anzeige jedes der Vielzahl von gefärbten Bildern (9) geändert werden kann, und
wenn die Vergrößerung der Anzeige jedes gefärbten Bildes (9) geändert wird, eine Vergrößerung der Anzeige der mittleren Region (96b) auf jedem gefärbten Bild (9) auch zusammen mit der Vergrößerung der Anzeige jedes gefärbten Bildes (9) geändert wird.

8. Analyseunterstützungsverfahren nach einem der Ansprüche 1 bis 7, ferner umfassend:
zwischen den Operationen a) und c) (S11, S14), Durchführen einer Auflösungsreduktionsverarbeitung an jedem der Vielzahl von gefärbten Bildern (9) (S12).

9. Analyseunterstützungsverfahren nach Anspruch 8, wobei
in jedem der Vielzahl von gefärbten Bildern (9), die der Auflösungsreduktionsverarbeitung unterzogen wurden, Zellen (82a, 82b) in der Probe (91) einen durchschnittlichen Durchmesser von größer oder gleich einer Pixelgröße aufweisen.

10. Analyseunterstützungsverfahren nach einem der Ansprüche 1 bis 9, wobei
das trainierte Modell, das zum Erfassen des Gleichförmigkeitsniveaus in der Operation c) (S14) verwendet wird, unter der Vielzahl von gefärbten Bildern (9) gemeinsam ist.

11. Programm (39) zum Unterstützen der Analyse eines gefärbten Bildes (9) einer Probe (91),
wobei das Programm (39) von einem Computer ausgeführt wird, um:
a) eine Vielzahl von gefärbten Bildern (9) durch Anwenden einer Vielzahl von Typen von Färbeverfahren auf eine Probe (91) vorzubereiten, wobei die Vielzahl von gefärbten Bildern (9) jeweils der Vielzahl von Typen von Färbeverfahren entspricht (S11);
b) jedes gefärbte Bild (9) der Vielzahl von gefärbten Bildern (9) in eine Vielzahl von segmentierten Regionen (96) in einem vorbestimmten Segmentierungsmodus (S13) zu segmentieren;
c) ein Gleichförmigkeitsniveau in jedem gefärbten Bild (9) unter Verwendung eines trainierten Modells, das durch maschinelles Lernen erzeugt wird, zu erfassen, wobei das Gleichförmigkeitsniveau die Gleichförmigkeit eines gefärbten Zustands jeder der Vielzahl von segmentierten Regionen (96) angibt (S14); und
d) die Eignung jeder segmentierten Region (96) der Vielzahl von segmentierten Regionen (96) für ein vorbestimmtes Analyseverfahren gemäß dem Gleichförmigkeitsniveau in jedem der Vielzahl von gefärbten Bildern (9) zu bestimmen (S15), wobei
das Gleichförmigkeitsniveau in einem Lernbild (81), das zum Erzeugen des trainierten Modells verwendet wird, basierend auf Leuchtdichtewerten einer positiven Zelle (82b) und einer negativen Zelle (82a) in dem Lernbild (81) bestimmt wird.

12. Analyseunterstützungsvorrichtung (1) zum Unterstützen der Analyse eines gefärbten Bildes (9) einer Probe (91), wobei die Analyseunterstützungsvorrichtung umfasst:
einen Segmentierer (42), der jedes gefärbte Bild (9) einer Vielzahl von gefärbten Bildern (9) in eine Vielzahl von segmentierten Regionen (96) in einem vorbestimmten Segmentierungsmodus segmentiert, wobei die Vielzahl von gefärbten Bildern (9) durch Anwenden einer Vielzahl von Typen von Färbeverfahren auf eine Probe (91) erhalten wird, wobei die Vielzahl von gefärbten Bildern (9) jeweils der Vielzahl von Typen von Färbeverfahren entspricht;
einen Niveauerfasser (43), der ein Gleichförmigkeitsniveau in jedem gefärbten Bild (9) unter Verwendung eines trainierten Modells, das durch maschinelles Lernen erzeugt wird, erfasst, wobei das Gleichförmigkeitsniveau die Gleichförmigkeit eines gefärbten Zustands jeder der Vielzahl von segmentierten Regionen (96) angibt; und
einen Bestimmer (44), der die Eignung jeder segmentierten Region (96) der Vielzahl von segmentierten Regionen (96) für ein vorbestimmtes Analyseverfahren gemäß dem Gleichförmigkeitsniveau in jedem der Vielzahl von gefärbten Bildern (9) bestimmt, wobei
das Gleichförmigkeitsniveau in einem Lernbild (81), das zum Erzeugen des trainierten Modells verwendet wird, basierend auf Leuchtdichtewerten einer positiven Zelle (82b) und einer negativen Zelle (82a) in dem Lernbild (81) bestimmt wird.

## Revendications

1. Procédé d'assistance à l'analyse destiné à faciliter l'analyse d'une image colorée (9) d'un échantillon (91), le procédé d'assistance à l'analyse comprenant :
a) préparer une pluralité d'images colorées (9) en appliquant une pluralité de types de procédés de coloration sur un échantillon (91), ladite pluralité d'images colorées (9) correspondant respectivement à ladite pluralité de procédés de coloration (S11) ;
b) segmenter chaque image colorée (9) de ladite pluralité d'images colorées (9) en une pluralité de régions segmentées (96) dans un mode de segmentation prédéterminé (S13) ;
c) acquérir un niveau d'uniformité dans chaque dite image colorée (9) en utilisant un modèle entraîné par apprentissage machine, le niveau d'uniformité indiquant l'uniformité d'une condition de coloration de chacune de ladite pluralité de régions segmentées (96) (S14) ; et
d) déterminer la convenance de chaque région segmentée (96) de ladite pluralité de régions segmentées (96) à un procédé d'analyse prédéterminé conformément audit niveau d'uniformité de ladite pluralité d'images colorées (9) (S15), dans lequel
ledit niveau d'uniformité dans une image d'apprentissage (81) qui est utilisée pour générer ledit modèle entraîné est déterminé sur la base de valeurs de luminance d'une cellule positive (82b) et d'une cellule négative (82a) dans ladite image d'apprentissage (81).

2. Procédé d'assistance à l'analyse selon la revendication 1, dans lequel
une région correspondant à l'analyse (96a) est affichée sur ladite pluralité d'images colorées (9) conformément à un résultat de la détermination dans ladite opération d) (S15), la région correspondant à l'analyse (96a) étant une région segmentée (96) qui correspond audit procédé d'analyse.

3. Procédé d'assistance à l'analyse selon la revendication 2, dans lequel
un grossissement de l'affichage de chacune de ladite pluralité d'images colorées (9) peut être changé, et
lorsque le grossissement de l'affichage de chaque dite image colorée (9) est changé, un grossissement de l'affichage de ladite région correspondant à l'analyse (96a) de chaque dite image colorée (9) est aussi changé avec le grossissement de l'affichage de chaque dite image colorée (9).

4. Procédé d'assistance à l'analyse selon l'une quelconque des revendications 1 à 3, dans lequel
une région ne correspondant pas à l'analyse (96c) est affichée sur ladite pluralité d'images colorées (9) conformément à un résultat de la détermination dans ladite opération d) (S15), la région ne correspondant pas à l'analyse (96c) étant une région segmentée (96) qui ne correspond pas audit procédé d'analyse.

5. Procédé d'assistance à l'analyse selon la revendication 4, dans lequel
un grossissement de l'affichage de chacune de ladite pluralité d'images colorées (9) peut être changé, et
lorsque le grossissement de l'affichage de chaque dite image colorée (9) est changé, un grossissement de l'affichage de ladite région ne correspondant pas à l'analyse (96c) sur chaque dite image colorée (9) est aussi changé avec le grossissement de chaque dite image colorée (9).

6. Procédé d'assistance à l'analyse selon l'une quelconque des revendications 1 à 5, dans lequel
une région centrale (96b) est affichée sur ladite pluralité d'images colorées (9) conformément à un résultat de la détermination dans ladite opération d) (S15), la région centrale (96b) étant une région segmentée (96) qui peut corresponde ou non audit procédé d'analyse.

7. Procédé d'assistance à l'analyse selon la revendication 6, dans lequel
un grossissement de l'affichage de chacune de ladite pluralité d'images colorées (9) peut être changé, et
lorsque le grossissement de l'affichage de chaque dite image colorée (9) est changé, un grossissement de l'affichage de ladite région centrale (96b) sur chaque dite image colorée (9) est aussi changé avec le grossissement de l'affichage de chaque dite image colorée (9).

8. Procédé d'assistance à l'analyse selon l'une quelconque des revendications 1 à 7, comprenant en outre :
entre lesdites opérations a) et c) (S11, S14), effectuer un traitement de réduction de résolution sur chacune de ladite pluralité d'images colorées (9) (S12).

9. Procédé d'assistance à l'analyse selon la revendication 8, dans lequel
dans chacune de ladite pluralité d'images colorées (9) qui ont subi ledit traitement de réduction de résolution, des cellules (82a, 82b) dans ledit échantillon (91) ont un diamètre moyen plus grand que ou égal à une taille de pixel.

10. Procédé d'assistance à l'analyse selon l'une quelconque des revendications 1 à 9, dans lequel
ledit modèle entraîné utilisé pour acquérir ledit niveau d'uniformité dans ladite opération c) (S14) est commun parmi ladite pluralité d'images colorées (9).

11. Programme (39) d'assistance à l'analyse d'une image colorée (9) d'un échantillon (91),
ledit programme (39) étant exécuté par un ordinateur pour :
a) préparer une pluralité d'images colorées (9) en appliquant une pluralité de types de procédés de coloration sur un échantillon (91), ladite pluralité d'images colorées (9) correspondant respectivement à ladite pluralité de types de procédés de coloration (S11) ;
b) segmenter chaque image colorée (9) de ladite pluralité d'images colorées (9) en une pluralité de régions segmentées (96) dans un mode de segmentation prédéterminé (S13) ;
c) acquérir un niveau d'uniformité dans chaque dite image colorée (9) en utilisant un modèle entraîné généré par apprentissage machine, le niveau d'uniformité indiquant l'uniformité d'une condition de coloration de chacune de ladite pluralité de régions segmentées (96) (S14) ; et
d) déterminer la convenance de chaque région segmentée (96) de ladite pluralité de régions segmentées (96) à un procédé d'analyse prédéterminé conformément audit niveau d'uniformité dans chacune de ladite pluralité d'images colorées (9) (S15), dans lequel
ledit niveau d'uniformité dans une image d'apprentissage (81) qui est utilisée pour générer ledit modèle entraîné est déterminé sur la base de valeurs de luminance d'une cellule positive (82b) et d'une cellule négative (82a) dans ladite image d'apprentissage (81).

12. Dispositif d'assistance à l'analyse (1) destiné à faciliter l'analyse d'une image colorée (9) d'un échantillon (91), le dispositif de support à l'analyse comprenant :
un segmenteur (42) qui segmente chaque image colorée (9) d'une pluralité d'images colorées (9) en une pluralité de régions colorées (96) dans un mode de segmentation prédéterminé, ladite pluralité d'images colorées (9) étant obtenues en appliquant une pluralité de types de procédés de coloration sur un échantillon (91), ladite pluralité d'images colorées (9) correspondant respectivement à ladite pluralité de types de procédés de coloration ;
un acquéreur de niveau (43) qui acquiert un niveau d'uniformité dans chaque dite image colorée (9) en utilisant un modèle entraîné généré par apprentissage machine, le niveau d'uniformité indiquant l'uniformité d'une condition de coloration de chacune de ladite pluralité de régions segmentées (96) ; et
un déterminant (44) qui détermine la convenance de chaque région segmentée (96) de ladite pluralité de régions segmentées (96) à un procédé d'analyse prédéterminé conformément audit niveau d'uniformité dans chacune de ladite pluralité d'images colorées (9), dans lequel
ledit niveau d'uniformité dans une image d'apprentissage (81) qui est utilisée pour générer ledit modèle entraîné est déterminé sur la base de valeurs de luminance d'une cellule positive (82b) et d'une cellule négative (82a) dans ladite image d'apprentissage (81).
